# EUROPÄISCHE PATENTSCHRIFT

(11) **EP 1 458 299 B1**
(45) Veröffentlichungstag und Bekanntmachung des Hinweises auf die Patenterteilung: **12.10.2005**
(21) Anmeldenummer: 01275091.5
(22) Anmeldetag: 24.12.2001
(51) Int. Cl.: A61B 17/80

(54) **VORRICHTUNG FÜR DIE OSTEOSYNTHESE**
DEVICE FOR PERFORMING OSTEOSYNTHESIS
DISPOSITIF POUR OSTEOSYNTHESE

(43) Veröffentlichungstag der Anmeldung: 22.09.2004
(73) Patentinhaber: Synthes AG Chur, 7002 Chur (CH)
(72) Erfinder: MATHIEU, Claude, CH-2544 Bettlach (CH); FRIGG, Robert, CH-2544 Bettlach (CH); SANER, Harald, CH-2545 Selzach (CH)
(74) Vertreter: Lusuardi, Werther Giovanni
(86) Internationale Anmeldenummer: PCT/CH2001/000740
(87) Internationale Veröffentlichungsnummer: WO 2003/055401

(56) Entgegenhaltungen:
- WO-A-99/59492
- FR-A- 2 674 118
- FR-A- 2 744 011
- US-B1- 6 235 033

## Beschreibung

Die Erfindung betrifft eine Vorrichtung für die Osteosynthese gemäss dem Oberbegriff des Patentanspruchs 1.

Solche Vorrichtungen werden zur polyaxialen, rigiden Verschraubung verwendet, insbesondere im Bereich der Wirbelsäule, beispielsweise für Pedikelschrauben oder Pedikelhaken. Sie können aber auch generell zur Plattenosteosynthese verwendet werden. Zudem sind auch Anwendungen für Fixateur externes sowie für Zwischenwirbleimplantate möglich.

Aus der US 6,235,033 (Basis für den Oberbegriff des Anspruch 1) ist bereits eine derartige Vorrichtung bekannt, bei der zwischen dem Schraubenkopf und der Bohrung der Knochenplatte ein schwenkbarer, ringförmiger Einsatz vorhanden ist, welcher mittels eines Schlitzes komprimierbar und expandierbar ist, um damit eine verbesserte Fixation zwischen Schraube und Platte zu erreichen. Der Nachteil dieser bekannten Vorrichtung besteht darin, dass der verwendete Einsatz kreisrund ausgebildet ist, so dass er beim Schraubeneindrehen mitdrehen kann und dadurch das Verriegeln verhindert. Der Einsatz kann sich aber auch ganz innerhalb der Plattenbohrung verdrehen, so dass er dann mit der falschen Seite (Innenkonus verjüngt sich in die falsche Richtung) nach oben zu liegen kommt.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung auch tatsächlich publiziert oder öffentlich bekannt war.

Hier will die Erfindung Abhilfe schaffen. Der Erfindung liegt das Problem zugrunde, eine Vorrichtung für die Osteosynthese zu schaffen, bei welcher die Knochenschrauben relativ zur Knochenplatte polyaxial beweglich und winkelstabil verriegelbar sind, ohne dass dazu zusätzliche mechanische Bauteile notwendig sind.

Die Erfindung löst die gestellte Aufgabe mit einer Vorrichtung, welche die Merkmale des Anspruchs 1 aufweist.

Als "unrund" wird im folgenden jeder von der exakt kreisförmigen Fläche abweichende Querschnitt bezeichnet, insbesondere prismatische und elliptische Querschnitte.

Damit ist der Vorteil erzielbar, dass sich der Einsatz beim Einschrauben der Knochenschraube nicht mehr um seine eigene Achse drehen kann. Letzteres würde nämlich dazu führen, dass keine Relativbewegung mehr zwischen Einsatz und Schraube stattfände, so dass auch kein Aufspreizen des Einsatzes mehr möglich wäre und damit auch keine Verriegelung der Schraube. Ein weiterer Vorteil besteht darin, dass keine zusätzliche Spreizschraube notwendig ist, wie dies bei der US 6,235,033 nötig ist.

Bei einer besonderen Ausführungsform ist der Querschnitt des Durchgangs in der vorzugsweise als Knochenplatte ausgebildeten osteosynthetischen Vorrichtung polygonal, vorzugsweise hexagonal, ausgebildet, so dass der Durchgang ein Prisma, vorzugsweise ein sechsseitiges Prisma darstellt. Bei der hexagonalen Ausführung lässt sich die Knochenschraube im sechskantigen Durchgang gleichzeitig in drei Ebenen schwenken, so dass jeder beliebige Schwenkwinkel einstellbar ist. Dieser ist nur durch die Plattendicke und durch das Anstossen des Einsatzes an der Querschnittsverengung begrenzt. Es können natürlich auch Knochenplatten mit mehreren Durchgängen verwendet werden.

Bei einer weiteren Ausführungsform ist der Durchmesser der zentralen Bohrung des Einsatzes in einer Richtung verjüngt und die Bohrung vorzugsweise als Konus augebildet. Diese Ausgestaltung erlaubt eine Aufspreizung des Einsatzes mittels eines Gegenkonus. Die Bohrung des Einsatzes kann aber auch kreiszylindrisch ausgebildet sein.

Vorzugsweise ist die Bohrung des Einsatzes mit einem Innengewinde versehen. Dies gestattet eine Verriegelung des Einsatzes.

Der orthogonal zur Zentralachse stehende Querschnitt des Durchgangs in der vorzugsweise als Knochenplatte ausgebildeten osteosynthetischen Vorrichtung kann auch ellipsenförmig ausgebildet sein.

Bei einer speziellen Ausführungsform besteht der Querschnitt des Durchgangs aus zwei unvollständigen Halbkreisen, welche mit nicht-kreisförmigen Linien verbunden sind. Der Einsatz weist dann auf seiner Aussenfläche entsprechend zwei Zapfen auf, welche in die durch die nicht-kreisförmigen Linien im Durchgang gebildeten Nuten einsetzbar sind.

Um den Einsatz radial komprimierbar und radial expandierbar zu gestalten, kann dieser einen durchgehenden, vorzugsweise parallel zur Längsachse des Einsatzes verlaufenden Schlitz aufweisen. Alternativ dazu kann der Einsatz auch mehrere, vorzugsweise parallel zur Längsachse verlaufende, nicht-durchgehende Schlitze aufweisen.

Die Oberfläche des Einsatzes, vorzugsweise im Bereich seiner peripheren Aussenfläche, ist zweckmässigerweise aufgerauht, z.B. rauh gestrahlt. Entsprechend kann der Durchgang in der Knochenplatte aufgerauht, z.B. rauh gestrahlt sein.

Die Oberfläche des Einsatzes kann aber auch, vorzugsweise im Bereich seiner peripheren Aussenfläche, mit einer Makrostrukturierung versehen sein, z.B. in Form peripher umlaufender Rillen. Der Durchgang kann dann in entsprechender Weise mit einer Makrostrukturierung versehen sein, z.B. in Form peripher umlaufender Rillen. Der Vorteil dieser Ausgestaltung liegt in der damit erzielten formschlüssigen Verbindung zwischen Einsatz und Knochenplatte.

Bei einer weiteren speziellen Ausführungsform verjüngt sich der Durchgang in der vorzugsweise als Knochenplatte ausgebildeten osteosynthetischen Vorrichtung gegen die Unterseite hin und vorzugsweise auch gegen die Oberseite hin, so dass eine Querschnittsverengung resultiert, welche das Herausfallen oder Herausdrücken des Einsatzes verhindert. Zweckmässigerweise werden dabei die Querschnittsverengung des Durchgangs und die Komprimierbarkeit des Einsatzes derart aufeinander abgestimmt, dass der Einsatz im komprimierten Zustand in den Durchgang einbringbar ist.

Die periphere Aussenfläche des Einsatzes ist zweckmässigerweise konvex, vorzugsweise zylinderförmig ausgebildet.

Vorteilhafterweise besteht die osteosynthetische Vorrichtung - mindestens im Bereich ihres Durchganges - und der Einsatz - ebenfalls mindestens im Bereich seiner peripheren Aussenfläche - aus verschiedenen Materialien, vorzugsweise solchen mit unterschiedlicher Härte. Der Einsatz kann beispielsweise aus einem biokompatiblen Kunststoff bestehen und die osteosynthetische Vorrichtung (z.B. eine Knochenplatte) aus einem körperverträglichen Metall. Der Einsatz kann auch aber metallisch sein und die Vorrichtung aus einem Kunststoff, vorzugsweise einem verstärkten Kunststoff. Die unterschiedlichen Materialien bewirken eine plastische Verformung der Oberflächen und dadurch einen Formschluss.

Die Höhe des Einsatzes sollte in Richtung seiner Längsachse gemessen, kleiner sein als die Höhe des Durchgangs in der Knochenplatte in Richtung seiner Zentralachse gemessen. Die Höhe des Einsatzes liegt zweckmässigerweise im Bereich von 40 % - 85 %, vorzugsweise von 45 % bis 65 % der Höhe des Durchgangs.

Die zur Einführung in den Einsatz dienenden Knochenschrauben weisen vorzugsweise einen konischen Schraubenkopf auf, der mit einem Aussengewinde versehen ist. Der Vorteil dieser Ausgestaltung liegt darin, dass das Spreizen und Verriegeln des Einsatzes in einem einzigen Schritt ermöglicht wird.

Die Erfindung und Weiterbildungen der Erfindung werden im folgenden anhand der teilweise schematischen Darstellungen mehrerer Ausführungsbeispiele noch näher erläutert. Alle Ausführungsbeispiele beziehen sich auf eine Knochenplatte als osteosynthetische Vorrichtung. Analoge Anwendungen für Pedikelschrauben, Pedikelhaken, Fixateur externes oder Zwischenwirbelimplantate sind möglich.

Es zeigen:
- Fig. 1: eine perspektivische Darstellung einer in Form einer Knochenplatte realisierten Vorrichtung für die Osteosynthese;
- Fig. 2: einen Querschnitt durch die Knochenplatte nach Fig. 1 mit einem eingesetzten Einsatz;
- Fig. 3: eine perspektivische Darstellung eines Einsatzes für die Vorrichtung für die Osteosynthese;
- Fig. 4: einen horizontalen Querschnitt durch den Einsatz nach Fig. 3;
- Fig. 5: eine perspektivische Darstellung eines Einsatzes für die Vorrichtung für die Osteosynthese;
- Fig. 6: einen horizontalen Querschnitt durch eine Variante der Knochenplatte;
- Fig. 7: eine perspektivische Darstellung eines Einsatzes für die Vorrichtung für die Osteosynthese passend zur Knochenplatte nach Fig. 6; und
- Fig. 8: einen Längsschnitt durch eine Knochenschraube für die Vorrichtung für die Osteosynthese.

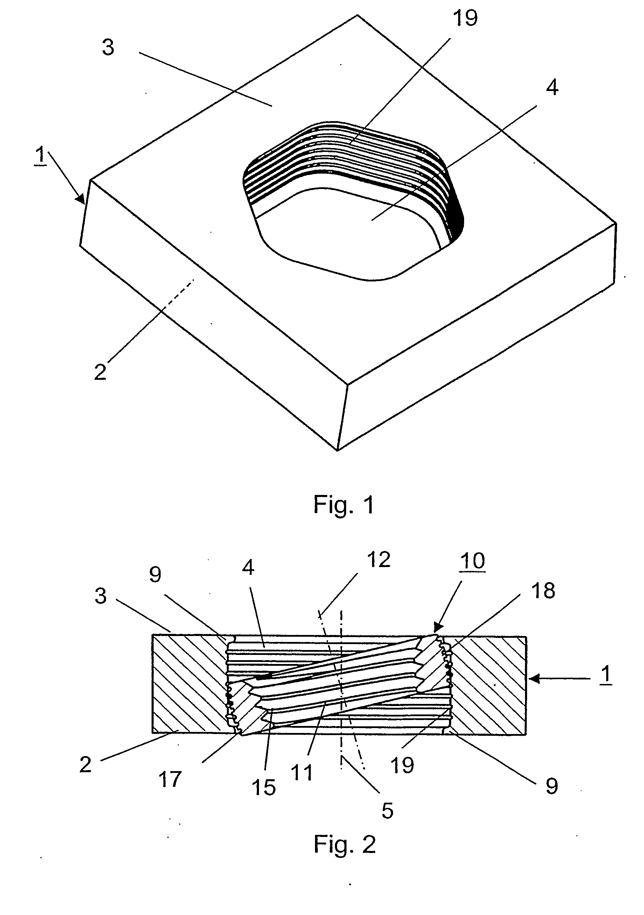

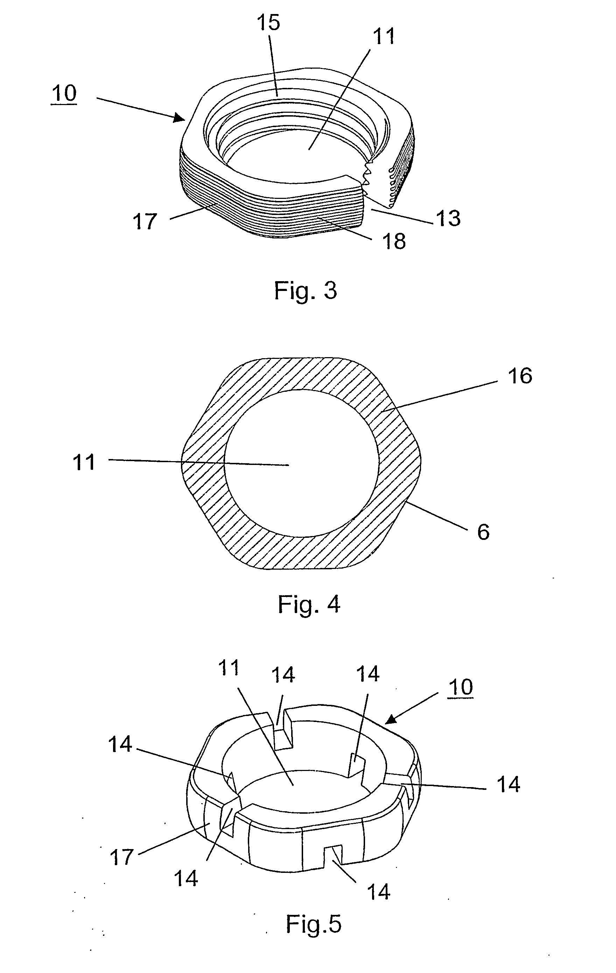

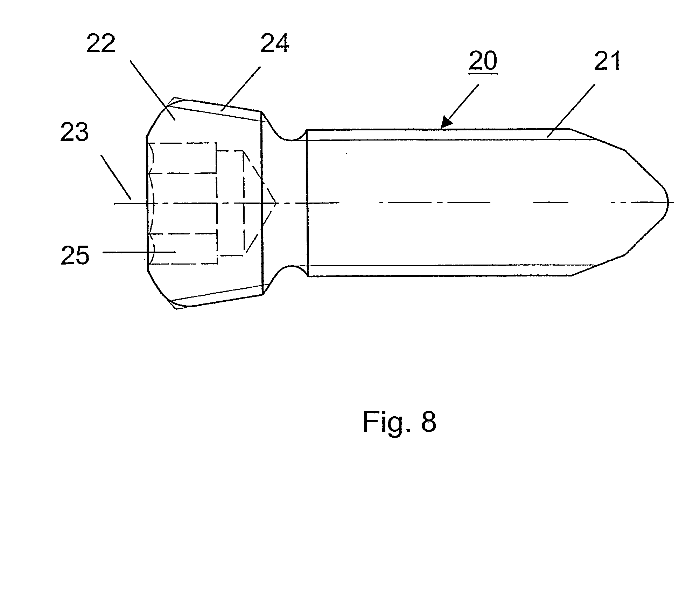

Die in den Fig. 1 bis 4 dargestellte Vorrichtung für die Osteosynthese besteht aus einer Knochenplatte 1 mit einer für die Anlage zum Knochen bestimmten Unterseite 2, einer Oberseite 3, sowie einem die Unterseite 2 mit der Oberseite 3 verbindenden Durchgang 4 zur Aufnahme eines multiaxial schwenkbaren Einsatzes 10 für eine Knochenschraube 20 (Fig. 8), wobei der Durchgang 4 eine Zentralachse 5 aufweist. Der in den Durchgang 4 einsetzbare Einsatz 10 (Fig. 3) besitzt eine zentrale Bohrung 11 zur Aufnahme der Knochenschraube 20 (Fig. 8), wobei die Bohrung 11 eine Längsachse 12 aufweist, sowie eine periphere Aussenfläche 17, welche für den Kontakt mit dem Durchgang 4 bestimmt ist.

Der Einsatz 10 weist einen durchgehenden Schlitz 13 auf, so dass er radial komprimierbar und radial expandierbar ist. Der Durchgang 4 der Knochenplatte 1 weist gegen die Unterseite 2 und auch gegen die Oberseite 3 hin, eine Querschnittsverengung 9 auf, um das Herausfallen oder Herausdrücken des Einsatzes 10 zu verhindern. Die Querschnittsverengung 9 des Durchgangs 4 und die Komprimierbarkeit des Einsatzes 10 sind derart aufeinander abgestimmt, dass der Einsatz 10 im komprimierten Zustand in den Durchgang 4 einbringbar ist.

Wie in Fig. 3 gezeigt, ist die Oberfläche des Einsatzes 10 im Bereich seiner peripheren Aussenfläche 17 mit einer Makrostrukturierung in Form von peripher umlaufenden Rillen 18 versehen. Entsprechend ist der Durchgang 4 der Knochenplatte 1 mit einer Makrostrukturierung in Form peripher umlaufender Rillen 19 (Fig. 2) versehen.

Wie in Fig. 4 gezeigt, ist der orthogonal zur Zentralachse 5 stehende Querschnitt 6 des Durchgangs 4 annähernd hexagonal, d.h. unrund ausgebildet ist. Der orthogonal zur Längsachse 12 stehende Querschnitt 16 des Einsatzes 10 weist eine zum Querschnitt 6 des Durchganges 4 der Knochenplatte 1 im wesentlichen korrespondierende Form auf, so dass der im Durchgang 4 eingesetzte Einsatz 10 relativ zu seiner Längsachse 12 rotationsfest ist, aber innerhalb des Durchgangs 4 relativ zur Knochenplatte 1 schwenkbar bleibt.
Wie in Fig. 2 dargestellt, verjüngt sich der Durchmesser der Bohrung 11 in Richtung der Unterseite 2 der Knochenplatte 1 hin, so dass die Bohrung 11 konisch augebildet ist. Die Bohrung 11 weist zudem ein Innengewinde 15 auf.

In Fig. 5 ist eine andere Ausführungsform des Einsatzes 10 dargestellt, der mehrere, parallel zur Längsachse 12 verlaufende, nicht-durchgehende Schlitze 14 aufweist. Dadurch wird der Einsatz 10 auch ohne durchgehenden Schlitz radial komprimierbar und radial expandierbar.

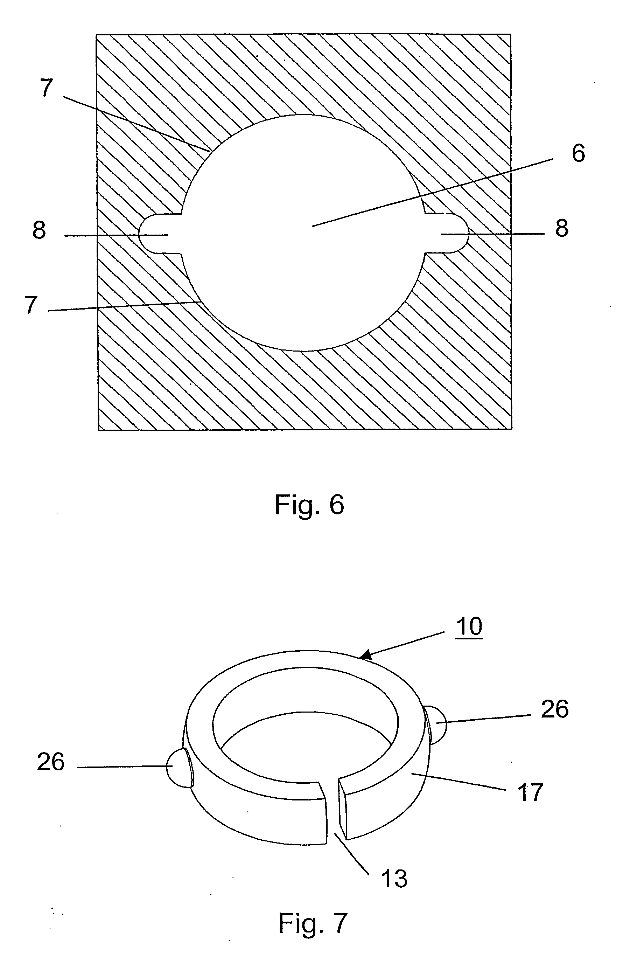

In den Fig. 6 und 7 ist eine andere Ausführungsform des Einsatzes 10 und der dazu korrespondierenden Knochenplatte 1 dargestellt, bei welcher der Querschnitt 6 des Durchganges 4 durch zwei unvollständige Halbkreise 7 definiert wird, welche durch zwei nicht-kreisförmige Linien 8 verbunden sind. Der in Fig. 7 dargestellte Einsatz weist dazu entsprechend einen Ring auf, dessen periphere Aussenfläche 17 kugelförmig ist und der zwei diametral angeordnete, halbkugelförmige Zapfen 26 aufweist. Die beiden Zapfen 26 finden Aufnahme in den von den nicht-kreisförmigen Linien 8 gebildeten Nuten im ebenfalls kugelförmigen Durchgang 4 der Knochenplatte 1. Der Einsatz 10 ist im eingesetzten Zustand sowohl um seine beiden Zapfen 26 als auch orthogonal zu dieser Drehachse drehbar, so dass alle Schwenkbewegungen möglich sind, ausser einer Verdrehung in der Plattenebene (Kardangelenk).

In den Einsatz 10 kann die in Fig. 8 dargestellte Knochenschraube 20 eingesetzt werden. Die Knochenschraube 20 besitzt einen zur Verankerung im Knochen bestimmten Gewindeschaft 21, eine Schraubenachse 23, sowie einen zur Einführung in die zentrale Bohrung 11 des Einsatzes 10 bestimmten Schraubenkopf 22, welcher im wesentlichen der Form der Bohrung 11 entspricht. Der orthogonal zur Schraubenachse 23 stehende Querschnitt durch den Schraubenkopf 22 ist gegen den Gewindeschaft 21 hin verjüngt, so dass ein Konus resultiert. Der Schraubenkopf 22 ist mit einem Aussengewinde 24 versehen, welches mit dem Innengewinde 15 des Einsatzes 10 korrespondiert. Im weiteren besitzt der Schraubenkopf 22 einen Innensechskant 15 zur Aufnahme eines (zeichnerisch nicht dargestellten) Sechskantschraubenziehers.

Im folgenden wird die klinische Verwendung der Vorrichtung für die Osteosynthese kurz beschrieben.
Der Einsatz 10 der Vorrichtung ist bereits in der Knochenplatte 1 oder Backe vormontiert. Ein Einsetzen durch den Chirurgen entfällt somit. Die Knochenplatte wird mit den vormontierten Einsätzen auf den Knochen gelegt. Dies kann entweder vor oder auch nach dem Reponieren der verschiedenen Bruchstücke, bzw. Wirbelkörper geschehen. Zum Setzen der Knochenschrauben gibt es drei Standard-Szenarien:
a) Bohren, Gewindeschneiden, Schrauben;
b) Bohren, Schrauben (mit selbstschneidenden Schrauben); oder
c) Schrauben (mit selbstbohrenden und selbstschneidenden Schrauben).

Auch die Verwendung von Ziel-, bzw. Bohrbuchsen ist möglich. Natürlich ist die Benützung von festen Zielbuchsen nicht sinnvoll, weil dadurch der Vorteil einer winkelverstellbaren Schraube verloren ginge, hingegen kann eine solche Zielbuchse sinnvoll sein, um den Verstellbereich einzuschränken. Bohrbuchsen kommen dann zum Einsatz wenn keine selbstbohrenden Schrauben verwendet werden und zuerst ein Loch gebohrt werden muss. In einem solchen Fall dient die Bohrbuchse dem Schutz der Weichteile.

Beim Setzen mehrerer Knochenschrauben gibt es grundsätzlich zwei Möglichkeiten:
A) Im Fall, dass die Reposition vor dem Anlegen der Platte gemacht wird, können die Schrauben sofort fixiert werden; und
B) für den Fall, dass die Reposition nach dem Anlegen der Platte gemacht wird, werden die Schrauben nur soweit eingedreht, damit die Platte am Knochen fixiert ist; erst dann findet die endgültige Reposition oder Korrektur statt und die Schrauben können durch Weiterdrehen um wenige Winkelgrade blockiert werden.

## Patentansprüche

1. Vorrichtung für die Osteosynthese mit
A) einem Durchgang (4) zur Aufnahme eines multiaxial schwenkbaren Einsatzes (10) für eine Knochenschraube (20), wobei der Durchgang (4) eine Zentralachse (5) aufweist;
B) einem in den Durchgang (4) einsetzbaren Einsatz (10) mit einer zentralen Bohrung (11) zur Aufnahme einer Knochenschraube (20), wobei die Bohrung (11) eine Längsachse (12) aufweist, sowie einer peripheren Aussenfläche (17), welche zum Kontakt im Inneren des Durchgang (4) bestimmt istk, wobei
C) der Einsatz (10) radial komprimierbar und radial expandierbar ausgebildet ist, **dadurch gekennzeichnet, dass**
D) der orthogonal zur Zentralachse (5) stehende Querschnitt (6) des Durchgangs (4) unrund ausgebildet ist; und
E) der orthogonal zur Längsachse (12) stehende Querschnitt (16) des Einsatzes (10) eine zum Querschnitt (6) des Durchganges (4) im wesentlichen korrespondierende Form aufweist; so dass
F) der im Durchgang (4) eingesetzte Einsatz (10) relativ zu seiner Längsachse (12) rotationsfest ist, aber innerhalb des Durchgangs (4) relativ zur Vorrichtung für die Osteosynthese schwenkbar bleibt.

2. Vorrichtung nach Anspruch 1, **dadurch gekennzeichnet, dass** der Querschnitt (6) des Durchgangs (4) polygonal, vorzugsweise hexagonal, ausgebildet ist.

3. Vorrichtung nach Anspruch 1 oder 2, **dadurch gekennzeichnet, dass** der Durchgang (4) im wesentlichen prismatisch, vorzugsweise als sechsseitiges Prisma ausgebildet ist.

4. Vorrichtung nach einem der Ansprüche 1 bis 3, **dadurch gekennzeichnet, dass** der Durchgang (4) und der Einsatz (10) zahnradförmig ausgebildet sind.

5. Vorrichtung nach einem der Ansprüche 1 bis 4, **dadurch gekennzeichnet, dass** sich der Durchmesser der Bohrung (11) in einer Richtung verjüngt und die Bohrung (11) vorzugsweise als Konus augebildet ist.

6. Vorrichtung nach einem der Ansprüche 1 bis 4, **dadurch gekennzeichnet, dass** die Bohrung (11) des Einsatzes (10) kreiszylindrisch ausgebildet ist.

7. Vorrichtung nach einem der Ansprüche 1 bis 6, **dadurch gekennzeichnet, dass** die Bohrung (11) ein Innengewinde (15) aufweist.

8. Vorrichtung nach einem der Ansprüche 1 bis 5, **dadurch gekennzeichnet, dass** der Querschnitt (6) ellipsenförmig ausgebildet ist.

9. Vorrichtung nach einem der Ansprüche 1 bis 5, **dadurch gekennzeichnet, dass** der Querschnitt (6) durch zwei unvollständige Halbkreise (7) definiert wird, welche mit nicht-kreisförmigen Linien (8) verbunden sind.

10. Vorrichtung nach einem der Ansprüche 1 bis 9, **dadurch gekennzeichnet, dass** der Einsatz (10) einen durchgehenden Schlitz (13) aufweist.

11. Vorrichtung nach Anspruch 10, **dadurch gekennzeichnet, dass** der Schlitz (13) parallel zur Längsachse (12) verläuft.

12. Vorrichtung nach Anspruch 10, dass der Einsatz (10) mehrere, vorzugsweise parallel zur Längsachse (12) verlaufende, nicht-durchgehende Schlitze (14) aufweist.

13. Vorrichtung nach einem der Ansprüche 1 bis 12, **dadurch gekennzeichnet, dass** die Oberfläche des Einsatzes (10), vorzugsweise im Bereich seiner peripheren Aussenfläche (17), aufgerauht ist, vorzugsweise rauh gestrahlt ist.

14. Vorrichtung nach einem der Ansprüche 1 bis 13, **dadurch gekennzeichnet, dass** die Oberfläche des Einsatzes (10), vorzugsweise im Bereich seiner peripheren Aussenfläche (17), mit einer Beschichtung aus einem härteren Material als der Einsatz (10) versehen ist.

15. Vorrichtung nach einem der Ansprüche 1 bis 14, **dadurch gekennzeichnet, dass** die Oberfläche des Einsatzes (10), vorzugsweise im Bereich seiner peripheren Aussenfläche (17), mit einer Makrostrukturierung versehen ist, vorzugsweise mit peripher umlaufenden Rillen (18).

16. Vorrichtung nach einem der Ansprüche 1 bis 15, **dadurch gekennzeichnet, dass** der Durchgang (4) aufgerauht ist, vorzugsweise rauh gestrahlt ist.

17. Vorrichtung nach einem der Ansprüche 1 bis 15, **dadurch gekennzeichnet, dass** der Durchgang (4) mit einer Beschichtung aus einem härteren Material als die Vorrichtung versehen ist.

18. Vorrichtung nach einem der Ansprüche 1 bis 17, **dadurch gekennzeichnet, dass** der Durchgang (4) mit einer Makrostrukturierung versehen ist, vorzugsweise mit peripher umlaufenden Rillen (19).

19. Vorrichtung nach einem der Ansprüche 1 bis 18, **dadurch gekennzeichnet, dass** der Durchgang (4) gegen die Unterseite (2) hin und vorzugsweise auch gegen die Oberseite (3) hin, eine Querschnittsverengung (9) aufweist, um das Herausfallen oder Herausdrücken des Einsatzes (10) zu verhindern.

20. Vorrichtung nach Anspruch 10 und 19, **dadurch gekennzeichnet, dass** die Querschnittsverengung (9) des Durchgangs (4) und die Komprimierbarkeit des Einsatzes (10) derart aufeinander abgestimmt ist, dass der Einsatz (10) im komprimierten Zustand in den Durchgang (4) einbringbar ist.

21. Vorrichtung nach einem der Ansprüche 1 bis 20, **dadurch gekennzeichnet, dass** die periphere Aussenfläche (17) des Einsatzes (10) konvex ausgebildet ist.

22. Vorrichtung nach einem der Ansprüche 1 bis 20, **dadurch gekennzeichnet, dass** die innere Fläche des Durchgangs (4) konkav und die periphere Aussenfläche (17) des Einsatzes (10) prismatisch ausgebildet ist.

23. Vorrichtung nach einem der Ansprüche 1 bis 22, **dadurch gekennzeichnet, dass** sie, mindesten im Bereich ihres Durchganges (4) und der Einsatz (10), mindestens im Bereich seiner peripheren Aussenfläche (17) aus verschiedenen Materialien, vorzugsweise solchen mit unterschiedlicher Härte, bestehen.

24. Vorrichtung nach einem der Ansprüche 1 bis 23, **dadurch gekennzeichnet, dass** sie aus einem Kunststoff, vorzugsweise einem verstärkten Kunststoff besteht und der Einsatz (10) metallisch ist.

25. Vorrichtung nach einem der Ansprüche 1 bis 24, **dadurch gekennzeichnet, dass** die Höhe des Einsatzes (10) in Richtung seiner Längsachse (12) gemessen, kleiner ist als die Höhe des Durchgangs (4) in Richtung seiner Zentralachse (5) gemessen.

26. Vorrichtung nach Anspruch 25, **dadurch gekennzeichnet, dass** die Höhe des Einsatzes (10) im Bereich von 40 % - 85 %, vorzugsweise von 45 % bis 65 % der Höhe des Durchgangs (4) liegt.

27. Vorrichtung nach einem der Ansprüche 1 bis 26, **dadurch gekennzeichnet, dass** sie eine Knochenschraube (20) mit einem zur Verankerung im Knochen bestimmten Gewindeschaft (21), einer Schraubenachse (23), und einem zur Einführung in die zentrale Bohrung (11) des Einsatzes (10) bestimmten Schraubenkopf (22), welcher im wesentlichen der Form der Bohrung (11) entspricht.

28. Vorrichtung nach Anspruch 27, **dadurch gekennzeichnet, dass** sich der orthogonal zur Schraubenachse (23) stehende Querschnitt durch den Schraubenkopf (22) gegen den Gewindeschaft (21) hin verjüngt, vorzugsweise in Form eines Konus.

29. Vorrichtung nach Anspruch 27 oder 28, **dadurch gekennzeichnet, dass** der Schraubenkopf (22) mit einem Aussengewinde (24) versehen ist.

30. Vorrichtung nach einem der Ansprüche 1 bis 29, **dadurch gekennzeichnet, dass** sie eine Knochenplatte (1) ist, mit einer für die Anlage zum Knochen bestimmten Unterseite (2), einer Oberseite (3) und mindestens einem die Unterseite (2) mit der Oberseite (3) verbindenden Durchgang (4).

31. Vorrichtung nach einem der Ansprüche 1 bis 29, **dadurch gekennzeichnet, dass** sie eine Pedikelschraube oder ein Pedikelhaken ist.

32. Vorrichtung nach einem der Ansprüche 1 bis 29, **dadurch gekennzeichnet, dass** sie ein Fixateur externe ist.

33. Vorrichtung nach einem der Ansprüche 1 bis 29, **dadurch gekennzeichnet, dass** sie ein Zwischenwirbelimplantat ist.

## Claims

1. A device for osteosynthesis, including
A) a through hole (4) designed to receive a multiaxially adjustable bushing (10) for a bone screw (20), the through hole (4) having a central axis (5);
B) a bushing (10) insertable in said through hole (4) including a central bore (11) designed to receive a bone screw (20), the bore (11) having a longitudinal axis (12), as well as a peripheral outside face (17) designed to be in contact with the interior of the through hole (4), whereby
C) the bushing (10) is realised in such a way as to be radially compressible and radially expansible;
**characterized in that**
D) the cross section (6) extending orthogonally to the central axis (5) is of non-circular shape; and
E) the cross section of the bushing (10) extending orthogonally to the longitudinal axis (12) is shaped in a form which substantially corresponds to that of the cross section (6) of the through hole (4); so that
F) the bushing (10) inserted in the through hole (4) is secured against rotation relative to its longitudinal axis (12) while remaining pivotable within the through hole (4) relative to the device for osteosynthesis.

2. A device as claimed in claim 1, **characterised in that** the cross section (6) of the through hole (4) is shaped in a polygonal, preferably a hexagonal form.

3. A device as claimed in claim 1 or 2, **characterised in that** the through hole (4) is shaped in the form of a prism, preferably a hexagonal prism.

4. A device as claimed in any of the claims 1 to 3, **characterised in that** the through hole (4) and the bushing (10) are shaped in a form resembling a toothed wheel.

5. A device as claimed in any of the claims 1 to 4, **characterised in that** the diameter of the bore (11) tapers in one direction and that the bore (11) is preferably shaped in the form of a cone.

6. A device as claimed in any of the claims 1 to 4, **characterised in that** the bore (11) of the bushing (10) is shaped in a circular cylindrical form.

7. A device as claimed in any of the claims 1 to 6, **characterised in that** the bore (11) has an internal screw thread (15).

8. A device as claimed in any of the claims 1 to 5, **characterised in that** the cross section (6) has an elliptical form.

9. A device as claimed in any of the claims 1 to 5, **characterised in that** the cross section (6) is defined by two incomplete semicircles (7) connected to one another by means of the non-circular lines (8).

10. A device as claimed in any of the claims 1 to 9, **characterised in that** the bushing (10) has a continuous slot (13).

11. A device as claimed in claim 10, **characterised in that** the slot (13) extends parallel to the longitudinal axis (12).

12. A device as claimed in claim 10, **characterised in that** the bushing (10) includes a plurality of non-continuous slots (14) preferably extending parallel to the longitudinal axis (12).

13. A device as claimed in any of the claims 1 to 12, **characterised in that** the surface of the bushing (10), preferably in the area of its peripheral, outside face (17), is roughened, preferably by means of grit blasting.

14. A device as claimed in any of the claims 1 to 13, **characterised in that** the surface of the bushing (10), preferably in the area of its peripheral, outside face (17), is provided with a coating made of a harder material than that of the bushing (10).

15. A device as claimed in any of the claims 1 to 14, **characterised in that** the surface of the bushing (10), preferably in the area of its peripheral, outside face (17), is provided with a macrostructured portion, preferably with peripheral ridges (18).

16. A device as claimed in any of the claims 1 to 15, **characterised in that** the through hole (4) is roughened, preferably by means of grit blasting.

17. A device as claimed in any of the claims 1 to 15, **characterised in that** the through hole (4) is provided with a coating made of a harder material than that of the osteosynthetic device.

18. A device as claimed in any of the claims 1 to 17, **characterised in that** the through hole (4) is provided with a macrostructured portion, preferably with peripheral ridges (19).

19. A device as claimed in any of the claims 1 to 18, **characterised in that** the through hole (4) is provided, towards the bottom surface (2) and preferably also towards the top surface (3), with a reduced cross section (9) designed to prevent the bushing (10) from falling out or from being pressed out.

20. A device as claimed in claims 10 and 19, **characterised in that** the reduced cross section (9) of the through hole (4) and the compressibility of the bushing (10) are adequately adapted to one another so that it is still possible to introduce the compressed bushing (10) into the through hole (4).

21. A device as claimed in any of the claims 1 to 20, **characterised in that** the peripheral outside face (17) of the bushing (10) is shaped in a convex form.

22. A device as claimed in any of the claims 1 to 20, **characterised in that** the inner surface of the through hole (4) is concave and that the peripheral outside face (17) of the bushing (10) is prismatic.

23. A device as claimed in any of the claims 1 to 22, **characterised in that** the osteosynthetic device, at least in the area of its through hole (4), and the bushing (10), at least in the area of its peripheral, outside face (17), consist of different materials, said materials being preferably of different hardness.

24. A device as claimed in any of the claims 1 to 23, **characterised in that** the osteosynthetic device consists of a plastic material, preferably a reinforced plastic material, and that the bushing (10) is metallic.

25. A device as claimed in any of the claims 1 to 14, **characterised in that** the height of the bushing (10) measured in the direction of its longitudinal axis (12) is inferior to the height of the through hole (4) measured in the direction of its central axis (5).

26. A device as claimed in claim 25, **characterised in that** the height of the bushing (10) is between 40 and 85 percent, preferably between 45 and 65 percent of the height of the through hole (4).

27. A device as claimed in any of the claims 1 to 26, **characterised in that** it comprises a bone screw (20) having a threaded shaft (21) for anchoring within the bone, a screw axis (23), and a screw head (22) designed to be introduced into the central bore (11) of the bushing (10), said screw head corresponding substantially to the form of the bore (11).

28. A device as claimed in claim 27, **characterised in that** the cross section of the screw head (22) extending orthogonally to the screw axis (23) tapers towards the threaded shaft (21), said taper being preferably conical.

29. A device as claimed in claim 27 or 28, **characterised in that** the screw head (22) is provided with an external screw thread (24).

30. A device as claimed in any of the claims 1 to 29, **characterised in that** the osteosynthesis device is a bone plate (1) including a bottom surface (2) designed to bear against the bone, a top surface (3), and at least one through hole (4) connecting the bottom surface (2) with the top surface (3).

31. A device as claimed in any of the claims 1 to 29, **characterised in that** the osteosynthesis device is a pedicle screw or a pedicle hook.

32. A device as claimed in any of the claims 1 to 29, **characterised in that** the osteosynthesis device is an external fixator.

33. A device as claimed in any of the claims 1 to 29, **characterised in that** the osteosynthesis device is an intervertebral implant.

## Revendications

1. Dispositif pour ostéosynthèse comportant
A) un trou de passage (4) permettant de recevoir un insert (10) pouvant pivoter de manière multiaxiale et destiné à recevoir une vis d'ostéosynthèse (20), le trou de passage (4) présentant un axe central (5);
B) un insert (10) pouvant être inséré dans le trou de passage (4) et comportant un trou central (11) permettant de recevoir une vis d'ostéosynthèse (20), le trou (11) présentant un axe longitudinal (12), ainsi qu'une surface extérieure périphérique (17) destinée à être en contact avec l'intérieur du trou de passage (4),
C) l'insert (10) pouvant être comprimé de manière radiale et élargi de manière radiale, **caractérisé en ce que**
D) la section transversale (6) du trou de passage (4), s'étendant orthogonalement à l'axe central (5), est de forme non-circulaire; et
E) la section transversale (16) de l'insert (10), s'étendant orthogonalement à l'axe longitudinal (12), présente une forme correspondant essentiellement à la section transversale (6) du trou de passage (4); de sorte que
F) l'insert (10) inséré dans le trou de passage (4) ne peut pas tourner par rapport à son axe longitudinal (12) mais continue à pouvoir pivoter, à l'intérieur dudit trou de passage (4), par rapport au dispositif pour ostéosynthèse.

2. Dispositif selon la revendication 1, **caractérisé en ce que** la section transversale (6) du trou de passage (4) est de forme polygonale, de préférence de forme hexagonale.

3. Dispositif selon la revendication 1 ou 2, **caractérisé en ce que** le trou de passage (4) présente essentiellement la forme d'un prisme, de préférence la forme d'un prisme hexagonal.

4. Dispositif selon l'une des revendications 1 à 3, **caractérisé en ce que** le trou de passage (4) et l'insert (10) présentent la forme d'une roue dentée.

5. Dispositif selon l'une des revendications 1 à 4, **caractérisé en ce que** le diamètre du trou (11) va en diminuant dans une direction donnée et que le trou (11) présente de préférence la forme d'un cône.

6. Dispositif selon l'une des revendications 1 à 4, **caractérisé en ce que** le trou (11) de l'insert (10) présente la forme d'un cylindre circulaire.

7. Dispositif selon l'une des revendications 1 à 6, **caractérisé en ce que** le trou (11) présente un filetage intérieur (15).

8. Dispositif selon l'une des revendications 1 à 5, **caractérisé en ce que** la section transversale (6) est réalisée sous forme d'ellipse.

9. Dispositif selon l'une des revendications 1 à 5, **caractérisé en ce que** la section transversale (6) est définie par deux demi-cercles incomplets reliés par des lignes non-circulaires (8).

10. Dispositif selon l'une des revendications 1 à 9, **caractérisé en ce que** l'insert (10) présente une fente traversante (13).

11. Dispositif selon la revendication 10, **caractérisé en ce que** la fente (13) s'étend parallèlement à l'axe longitudinal (12).

12. Dispositif selon la revendication 10, **caractérisé en ce que** l'insert (10) présente plusieurs fentes non-traversantes (14), s'étendant de préférence parallèlement à l'axe longitudinal (12).

13. Dispositif selon l'une des revendications 1 à 12, **caractérisé en ce que** la surface de l'insert (10) est rugueuse, de préférence traitée par sablage, et ce de préférence dans la zone de sa surface extérieure périphérique (17).

14. Dispositif selon l'une des revendications 1 à 13, **caractérisé en ce que** la surface de l'insert (10) est pourvue, de préférence dans la zone de sa surface extérieure périphérique (17), d'un revêtement réalisé en un matériau plus dur que l'insert (10).

15. Dispositif selon l'une des revendications 1 à 14, **caractérisé en ce que** la surface de l'insert (10) est pourvue, de préférence dans la zone de sa surface extérieure périphérique (17), d'une macrostructure, de préférence de rainures circonférentielles (18).

16. Dispositif selon l'une des revendications 1 à 15, **caractérisé en ce que** le trou de passage (4) est rugueux, de préférence traité par sablage.

17. Dispositif selon l'une des revendications 1 à 15, **caractérisé en ce que** le trou de passage (4) est pourvu d'un revêtement réalisé en un matériau plus dur que le dispositif.

18. Dispositif selon l'une des revendications 1 à 17, **caractérisé en ce que** le trou de passage (4) est pourvu d'une macrostructure, de préférence de rainures circonférentielles (19).

19. Dispositif selon l'une des revendications 1 à 18, **caractérisé en ce que** le trou de passage (4) présente, vers la face inférieure (2) et de préférence aussi vers la face supérieure (3), un rétrécissement (9) de la section transversale afin d'éviter que l'insert (10) ne sorte par le haut ou par le bas.

20. Dispositif selon les revendications 10 et 19, **caractérisé en ce que** le rétrécissement (9) de la section transversale du trou de passage (4) et la compressibilité de l'insert (10) sont adaptés l'un à l'autre de telle sorte qu'il est possible d'insérer l'insert (10), à l'état comprimé, dans le trou de passage (4).

21. Dispositif selon l'une des revendications 1 à 20, **caractérisé en ce que** la surface extérieure périphérique (17) de l'insert (10) est de forme convexe.

22. Dispositif selon l'une des revendications 1 à 20, **caractérisé en ce que** la surface intérieure du trou de passage (4) est de forme concave et la surface extérieure périphérique (17) de l'insert (10) est de forme prismatique.

23. Dispositif selon l'une des revendications 1 à 22, **caractérisé en ce que** celui-ci est constitué, et ce au moins dans la zone de son trou de passage (4), et **en ce que** l'insert (10) est constitué, et ce au moins dans la zone de sa surface extérieure périphérique (17), en des matériaux différents, de préférence en des matériaux de dureté différente.

24. Dispositif selon l'une des revendications 1 à 23, **caractérisé en ce qu'**il est constitué par une matière plastique, de préférence par une matière plastique renforcée, et **en ce que** l'insert (10) est en métal.

25. Dispositif selon l'une des revendications 1 à 24, **caractérisé en ce que** la hauteur de l'insert (10), mesurée dans le sens de son axe longitudinal (12), est inférieure à la hauteur du trou de passage (4) mesurée dans le sens de son axe central (5).

26. Dispositif selon la revendication 25, **caractérisé en ce que** la hauteur de l'insert (10) est comprise entre 40 % et 85 %, de préférence entre 45 % et 65 % de la hauteur du trou de passage (4).

27. Dispositif selon l'une des revendications 1 à 26, **caractérisé en ce qu'**il comprend une vis d'ostéosynthèse (20) pourvue d'un corps fileté (21) destiné à être ancré dans l'os, un axe de vis (23) et une tête de vis (22) destinée à être insérée dans le trou central (11) de l'insert (10) et correspondant essentiellement à la forme du trou (11).

28. Dispositif selon la revendication 27, **caractérisé en ce que** la section transversale de la tête de vis (22), s'étendant orthogonalement à l'axe de vis (23), va en diminuant vers le corps fileté (21), de préférence sous la forme d'un cône.

29. Dispositif selon la revendication 27 ou 28, **caractérisé en ce que** la tête de vis (22) est pourvue d'un filetage extérieur (24).

30. Dispositif selon l'une des revendications 1 à 29, **caractérisé en ce que** celui-ci est une plaque d'ostéosynthèse (1) comportant une face inférieure (2) destinée à reposer contre l'os, une face supérieure (3) et au moins un trou de passage (4) reliant la face inférieure (2) à la face supérieure (3).

31. Dispositif selon l'une des revendications 1 à 29, **caractérisé en ce que** celui-ci est une vis pédiculaire ou un crochet pédiculaire.

32. Dispositif selon l'une des revendications 1 à 29, **caractérisé en ce que** celui-ci est un fixateur externe.

33. Dispositif selon l'une des revendications 1 à **caractérisé en ce que** celui-ci est un implant intervertébral.
